# EUROPÄISCHE PATENTANMELDUNG

(11) **EP 1 445 308 A1**
(43) Veröffentlichungstag der Anmeldung: **11.08.2004**
(21) Anmeldenummer: 03029264.3
(22) Anmeldetag: 20.12.2003
(51) Int. Cl.: C12M 1/22

(54) **Verfahren zum Abnehmen eines Deckels einer Petrischale und Deckelabnehmeinrichtung**

(30) Priorität: 09.01.2003 DE 10301447
(71) Anmelder: A.I.D. Autoimmun Diagnostika GmbH, 72479 Strassberg (DE)
(72) Erfinder: Mayer, Dietmar, 72336 Balingen (DE); Schöllhorn, Volkmar, 72458 Albstadt-Ebingen (DE)
(74) Vertreter: Patentanwälte Ruff, Wilhelm, Beier, Dauster & Partner

(57) **Zusammenfassung**

Bei einem Verfahren zum Abnehmen eines Deckels einer Petrischale, wobei die Petrischale ein einen Boden und eine zylindrische Seitenwand aufweisendes schalenförmiges Unterteil besitzt und der einen nach unten weisende Deckelrand, die Seitenwand des Unterteils außen überlappende Deckel lose auf dem Unterteil aufliegt, wird zunächst der Deckelrand an seiner Unterkante an mindestens zwei gegenüberliegenden Seiten erfasst. Danach erfolgt eine seitliche Bewegung des Unterteils und des Deckels auf getrennten Führungen, die in Bewegungsrichtung unter relativem Anheben des Deckels in einem divergierenden Winkel zueinander verlaufen, wobei der Deckel an der in Bewegungsrichtung weisenden Seite relativ vom Unterteil unter Kippbewegung so weit angehoben wird, dass das Unterteil unter dem Deckel wegbewegbar ist. Danach erfolgt ein relatives Wegbewegen des Unterteils vom Deckel. Eine Deckelabnehmeinrichtung (14) zum Abnehmen eines Deckels (12d) einer Petrischale (12), wobei die Petrischale (12) ein einen Boden (12b) und eine zylindrische Seitenwand (12c) aufweisendes schalenförmiges Unterteil besitzt, und der einen nach unten weisenden Deckelrand (12d), die Seitenwand (12c) des Unterteils (12a) außen überlappende Deckel (12d) lose auf dem Unterteil (12a) aufliegt, besitzt eine (67) zum Anheben des Deckels (12d) vom Unterteil (12a) und eine Unterteilführung (66), die in Bewegungsrichtung und Anhebrichtung in einem divergierenden Winkel zueinander angeordnet sind.

## Beschreibung

Die Erfindung betrifft ein Verfahren und eine Deckelabnehmeinrichtung zum Abnehmen eines Deckels einer Petrischale. Die Petrischale besitzt ein einen Boden und einer zylindrischen Seitenrand aufweisendes schalenförmiges Unterteil und einen lose auf dem Unterteil aufliegenden Deckel mit einem nach unten weisenden, den Seitenrand des Unterteils außen überlappenden Deckelrand.

Bei vielen biologischen Untersuchungen werden Petrischalen als Kultivierungsgefäß für biologisches Material, insbesondere Zellmaterial eingesetzt. Das biologische Material befindet sich dabei auf einem Nährboden aus Nährmedium, insbesondere Aggar. Nach einer bestimmten Kultivierungsphase des biologischen Materials, beispielsweise einem Anwachsen von Bakterienkulturen, wird es untersucht. Dazu wird die Petrischale in eine Auswerteeinrichtung, beispielsweise in eine Bildauswerteeinrichtung befördert. Da in einem mikrobiologischen Labor viele Petrischalen mit auszuwertendem biologischem Material anfallen, besteht der Bedarf, dass der Transport zur bzw. von der Auswerteeinrichtung und die Auswertung im wesentlichen automatisiert erfolgt.

Vorzugsweise besitzen die in der Mikrobiologie eingesetzten Petrischalen ein Unterteil, dass das Nährmedium und die Probe aufnimmt sowie einen Deckel, der dazu vorgesehen ist, die durch biologisches Material verursachten Gerüche oder biologisches Material selber nicht in die Umgebung gelangen zu lassen. Zur Auswertung der Proben besteht jedoch die Notwendigkeit, die Petrischalen zu öffnen, also den Deckel abzunehmen, um beispielsweise bei einer Bildauswertung ein zuverlässiges Bild vom biologischen Material, insbesondere von Bakterienkulturen o.dgl. zu erhalten. Es ist natürlich möglich, den Deckel per Hand abzunehmen, dies ist jedoch bei einer Vielzahl anfallender Petrischalen nicht rationell.

### Aufgabe und Lösung

Aufgabe der Erfindung ist es, ein Verfahren und eine Deckelabnehmeinrichtung der eingangs erwähnten Art zu schaffen, die ein einfaches und rationelles Abnehmen des Deckels von der Petrischale ermöglichen.

Diese Aufgabe wird mit einem Verfahren mit den Merkmalen des unabhängigen Anspruchs 1 und einer Deckelabnehmeinrichtung mit den Merkmalen des unabhängigen Anspruchs 8 gelöst. Weiterbildungen der Erfindung sind in den Unteransprüchen dargestellt und werden im folgenden näher erläutert.

Das Verfahren zeichnet sich dadurch aus, dass zunächst der Deckelrand an seiner Unterkante an mindestens zwei gegenüberliegenden Seiten erfasst wird. Es folgt eine seitliche Bewegung des Unterteils und des Deckels auf getrennten Führungen, die in Bewegungsrichtung unter relativem Anheben des Deckels in einem divergierenden Winkel zueinander verlaufen, wobei der Deckel an der in Bewegungsrichtung weisenden Seite relativ vom Unterteil unter Kippbewegung so weit angehoben wird, dass das Unterteil unter dem Deckel wegbewegbar ist. Danach wird das Unterteil vom Deckel relativ wegbewegt.

Es wird also ein Verfahren geschaffen, mit dem sich in einfacher Weise der Deckel einer Petrischale abnehmen lässt. Es ist nicht erforderlich, den Deckel per Hand abzunehmen oder gar eine aufwendige Greifapparatur mit Deckelgreifern vorzusehen. Bei einer solchen Greifapparatur müsste zunächst aufwendig die Position der Petrischale und insbesondere des Deckels bestimmt werden, bevor der Deckel gegriffen und abgenommen werden kann. Nach dem Gebrauch der Petrischale, beispielsweise nach einer Auswertung, müsste wiederum die Position des Unterteils bestimmt werden, damit durch den Greifer der Deckel exakt auf das Unterteil gelegt werden kann. Dies ist beim erfindungsgemäßen Verfahren nicht notwendig.

Als Gebrauch der Petrischale im Sinne der Anmeldung wird insbesondere eine Auswertung des auf dem Nährboden befindlichen biologischen Materials verstanden. Beim biologischen, insbesondere mikrobiologischen Material handelt es sich vorzugsweise um Keime aus Zellmaterial, beispielsweise Bakterienkulturen. Die Auswertung kann beispielsweise eine Bildauswertung sein, bei der ein Schwarz-weiß- oder Farbbild vom biologischen Material gemacht wird. Beispielsweise kann aufgrund der Farbe von Keimen auf deren Identität geschlossen werden.

Die seitliche Bewegung mit relativem Anheben des Deckels vom Unterteil erfolgt vorzugsweise derart, dass der Deckel gegenüber der Horizontalen nach oben bewegt wird, während das Unterteil in Horizontalrichtung bewegt wird. Alternativ ist es möglich, dass der Deckel in Horizontalrichtung bewegt wird und das Unterteil gegenüber der Horizontalen nach unten bewegt wird. Eine weitere Alternative ist, den Deckel nach oben und das Unterteil gleichzeitig nach unten zu bewegen.

Beim seitlichen Bewegen des Deckels und des Unterteils wird der Deckel vorzugsweise mittels einer Deckelführung geführt und gegenüber dem Unterteil angehoben. Das Unterteil befindet sich dabei bevorzugt auf einer Unterteilführung. Die Deckelführung und die Unterteilführung können in Bewegungsrichtung und Anhebrichtung in einem divergierenden Winkel zueinander angeordnet sein. Die Unterteilführung greift vorzugsweise an der Unterseite des Petrischalen-Bodens an.

Die Deckelführung besitzt eine Deckeluntergreifungseinrichtung zur Erfassung und seitlichen Untergreifung des Deckelrands. Damit das Unterteil und der Deckel nicht unter der Deckelführung hindurchbewegt werden, ohne dass der Deckel erfasst wird, ist ein der Petrischale zugewandtes Ende der Deckeluntergreifungseinrichtung unterhalb einer Unterkante des Deckelrandes angeordnet.

Als Deckeluntergreifungseinrichtung sind vorzugsweise zwei Schienen vorgesehen, die im wesentlichen parallel zur Bewegungsrichtung beabstandet voneinander angeordnet sind. Damit der Deckel von der Deckeluntergreifungseinrichtung erfasst wird, ist es notwendig, dass der lichte Abstand der beiden Schienen kleiner ist als der Außendurchmesser des Deckels. Jedoch muss der lichte Abstand der beiden Schienen größer sein als der Außendurchmesser des Unterteils, damit dieses nicht bei seiner Bewegung in Bewegungsrichtung gebremst wird.

Befindet sich der Deckel auf der Deckelführung kann er zusätzlich durch eine Seitenführung seitlich geführt werden, so dass er beim relativen Anheben gegenüber dem Unterteil sozusagen in der Spur bleibt. Vorzugsweise ist die Seitenführung der Deckeluntergreifungseinrichtung zugeordnet. Es ist möglich, dass die Seitenführung ein separates Bauteil ist, das lediglich der Deckeluntergreifungseinrichtung zugeordnet ist. Besonders bevorzugt weist jedoch die Deckeluntergreifungseinrichtung selber eine Seitenführung auf, die insbesondere einstückig mit der Deckeluntergreifungseinrichtung verbunden ist.

Die Seitenführung kann zwei Führungselemente aufweisen, die parallel zur Bewegungsrichtung beabstandet voneinander angeordnet sind, wobei deren lichter Abstand im wesentlichen dem Außendurchmesser des Deckels entspricht. Dadurch kann der Deckel an der Seitenführung entlang gleitend geführt werden.-Es ist auch möglich, den lichten Abstand größer als den Außendurchmesser des Deckels auszubilden, so dass der Deckelrand lediglich auf der Deckeluntergreifungseinrichtung aufliegt und die Seitenführungen nicht berührt. Es ist auch möglich den lichten Abstand kleiner als den Außendurchmesser des Deckels auszubilden, beispielsweise wenn die Seitenführung elastisch federnd ausgebildet ist und sich beim Einfädeln des Deckels aufweitet.

Um ein exaktes Einfädeln des Deckels in die Deckelführung zu ermöglichen, sind vorzugsweise an den Führungselementen der Seitenführung Einlaufschrägen vorgesehen.

Bei einer Weiterbildung der Erfindung wird die Petrischale bei der seitlichen Bewegung am Deckel erfasst und vorzugsweise nur am Deckel seitlich verfahren, insbesondere verschoben. Durch die Erfassungsstelle am Deckel wird erreicht, dass der Deckelrand an der entgegen der Bewegungsrichtung weisenden hinteren Seite des Deckels auf den Seitenrand des Petrischalen-Unterteils gedrückt wird und dabei die Anhebung des Deckels gegenüber dem Unterteil unterstützt wird. Es kann somit eine Kipp- oder Schwenkachse zwischen Unterteil und Deckel geschaffen werden.

Ist ein bestimmter Öffnungswinkel zwischen dem Deckel und dem Unterteil erreicht, kann die Petrischale am Unterteil erfasst werden und am Unterteil seitlich verfahren, insbesondere verschoben werden. Es ist dadurch möglich, das Unterteil gegenüber dem Deckel weiterzubewegen, während der Deckel auf der Deckelführung liegen bleibt. Dies kann beispielsweise durch Umsetzen senkrecht zur Bewegungsrichtung erfolgen.

Bei einer Weiterbildung der Erfindung wird der Deckel nach einem bestimmten Weg auf der Deckelführung abgebremst oder schlägt an einen Anschlag an. Dazu ist vorzugsweise eine Bremse und/oder ein Anschlag an der Deckelführung vorgesehen. Die Bremse kann gleichzeitig als Niederhalter wirken, die den Deckel beim Anheben gegenüber dem Unterteil im wesentlichen senkrecht zur Bewegungsrichtung niederhält, so dass der Deckel nicht vorzeitig vom Unterteil abspringt und bei der Abkopplung von der Vorschubeinrichtung in seiner Lage verbleibt, was für die Wiedererfassung bei der Rückwärtsbewegung des Unterteils wichtig ist. Als bremsender Niederhalter kann beispielsweise ein schmaler Draht vorgesehen sein, der federnd vorzugsweise in der Mitte zwischen den Schienen der Deckeluntergreifungseinrichtung angeordnet ist. Der Niederhalter kann eine Einlaufschräge in Form einer Aufbiegung aufweisen, die beim Einfädeln des Deckels unter den Niederhalter hilft.

Bei einer Weiterbildung der Erfindung verbleibt der Deckel in einer solchen relativen Kipplage, dass das Unterteil bei einer Rückwärtsbewegung den Deckel mitnimmt, so dass dieser bei Rückgängigmachung der relativen Kipplage wieder auf dem Unterteil zur Ablage kommt.

Die Bremse und/oder der Anschlag können so angeordnet sein, dass in der Endlage des Deckels, der lichte Abstand zwischen der Deckeluntergreifungseinrichtung, an einer in Bewegungsrichtung hinteren Seite des Deckels und der Unterteilführung kleiner ist, als die Höhe des Seitenrandes des Unterteils. Anders ausgedrückt kann der lichte Abstand zwischen der Unterkante des Deckelrandes und der Lage des Petrischalenbodens kleiner sein als die Höhe des Seitenrandes des Unterteils. Dadurch ist es möglich, dass der Seitenrand des Petrischalen-Unterteils bei der Rückwärtsbewegung durch die gegenseitige Überlappung an die Innenseite des Deckelrandes anstößt und den Deckel wieder mitnimmt.

Zur seitlichen Bewegung der Petrischale kann mindestens ein Fahrgestell vorgesehen sein. Vorzugsweise sind drei Fahrgestelle vorgesehen, von denen ein Fahrgestell für die Bewegung in einer horizontalen x-Richtung dient, ein anderes Fahrgestell für die Bewegung in einer dazu senkrechten horizontalen z-Richtung und ein drittes Fahrgestell für die Bewegung in einer vertikalen y-Richtung. Dadurch ist es möglich, die Petrischale an jede beliebige Position innerhalb der Reichweite der Fahrgestelle zu verfahren.

Das Fahrgestell kann mit einem Schlitten zum Transport der Petrischale gekoppelt sein. Vorzugsweise ist dies das Fahrgestell zur Bewegung in der y-Richtung. Der Schlitten kann als Tandemschlitten ausgebildet sein, mit einem Support zur Aufnahme der Petrischale und einer Schiebeeinrichtung zum seitlichen Erfassen und Verschieben der Petrischale.

Der Support kann eine mittige Durchbrechung aufweisen. Bevorzugt ist der Support als relativ schmales Materialband ausgebildet, das eine äußere und eine die Durchbrechung definierende innere Kontur bildet.

Die Schiebeeinrichtung kann in Schieberichtung gegenüberliegende Randbegrenzungen, insbesondere Zentrierungen, aufweisen, deren lichter Abstand größer ist als der Außendurchmesser des Unterteils und kleiner ist als der Außendurchmesser des Deckels. Die Randbegrenzungen können auch senkrecht zur Schieberichtung liegen. Besonders bevorzugt sind Randbegrenzungen vorgesehen, die den Umfang der Petrischale, insbesondere den Deckel, umgreifen.

Im Bereich der Randbegrenzungen kann ein Vorsprung vorgesehen sein, der in Schieberichtung in den Abstandsbereich der Anschlagelemente hineinragt und die Erfassungsstelle zwischen der Schiebeeinrichtung und dem Deckel darstellt.

Es ist möglich, dass die Petrischale mittels des Supports unten aus einem Magazin herausgenommen wird und beim Absenken des Supports auf einen durch eine Öffnung des Supports den Boden der Petrischale erfassenden Tisch abgesetzt wird. Nach dem Absetzen der Petrischale auf dem Tisch kann diese durch die Schiebeeinrichtung übernommen und seitlich verschoben werden.

Das Unterteil kann nach Abnehmen des Deckels in eine Position zum Gebrauch, insbesondere zur Bildauswertung, gebracht werden und danach vorzugsweise wieder in Richtung zum Deckel bewegt werden, wobei der Deckel wieder aufgelegt wird. Dazu ist eine Gebrauchseinrichtung zum Gebrauch der Petrischale vorgesehen. Die Gebrauchseinrichtung kann eine Bildauswerteeinrichtung sein. Es sind auch andersartige Auswerteeinrichtungen möglich. Damit das Unterteil mit den zu untersuchenden Proben schnell von der Deckelabnehmeinrichtung zur Gebrauchseinrichtung gelangt, kann die Unterteilführung der Deckelabnehmeinrichtung zur Gebrauchseinrichtung hinführen.

Die Gebrauchseinrichtung ist bevorzugt eine Bildauswerteeinrichtung, insbesondere mit einer lichtdurchlässigen Photolichtplatte, einer Kamera und einer Auf- oder Durchlichteinheit.

Ferner umfasst die Erfindung eine Deckelabnehmeinrichtung mit den Merkmalen des unabhängigen Anspruchs 8.

Bezüglich näherer Details der Deckelabnehmeinrichtung wird auf die vorgehende Beschreibung und die nachfolgende Beschreibung von Ausführungsbeispielen in Verbindung mit der Zeichnung und den Unteransprüchen verwiesen.

Die vorstehenden und weiteren Merkmale der Erfindung gehen außer aus den Ansprüchen aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Die Unterteilung der Anmeldung in einzelne Abschnitte sowie Zwischenüberschriften beschränkt die unter diesen gemachten Aussagen nicht in ihrer Allgemeingültigkeit.

### Beschreibung der Zeichnungen

Bevorzugte Ausführungsbeispiele der Erfindung sind in den Zeichnungen dargestellt und werden im folgenden näher erläutert. In den Zeichnungen zeigen:
- Fig. 1: eine Innenansicht eines Automaten zum Petrischalen-Handling von oben her gesehen;
- Fig. 2: eine Vorderansicht eines Magazins einer erfindungsgemäßen Ent- und Beladevorrichtung für Petrischalen;

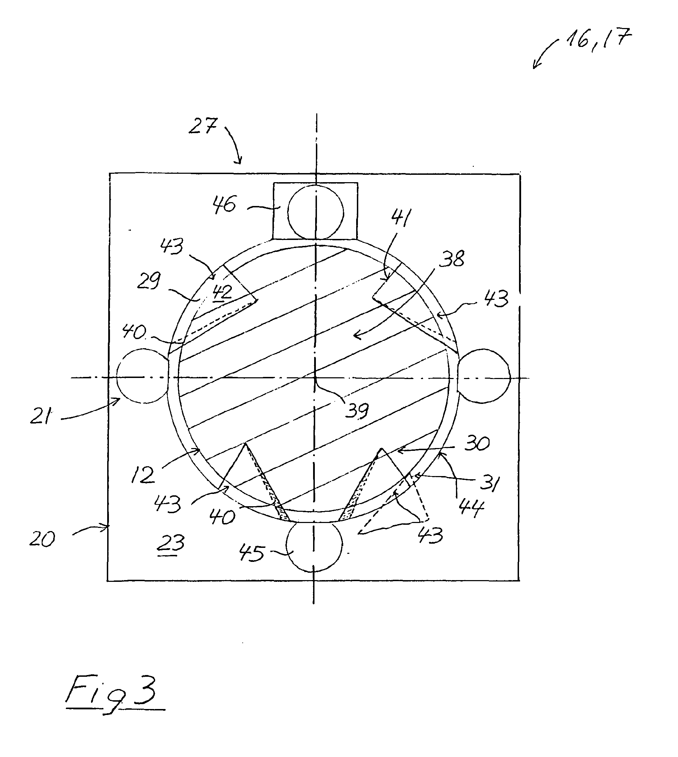

- Fig. 3: eine Draufsicht auf das Magazin von Fig. 2;
- Fig. 4: eine Unteransicht auf das Magazin von Fig. 2;
- Fig. 5: eine Draufsicht auf einen Tandemschlitten der Ent- und Beladevorrichtung;
- Fig. 6: eine Seitenansicht auf eine erfindungsgemäße Deckelabnehmeinrichtung für Petrischalen und
- Fig. 7: eine Draufsicht auf die Deckelabnehmeinrichtung nach Fig. 6.

### Detaillierte Beschreibung der Ausführungsbeispiele

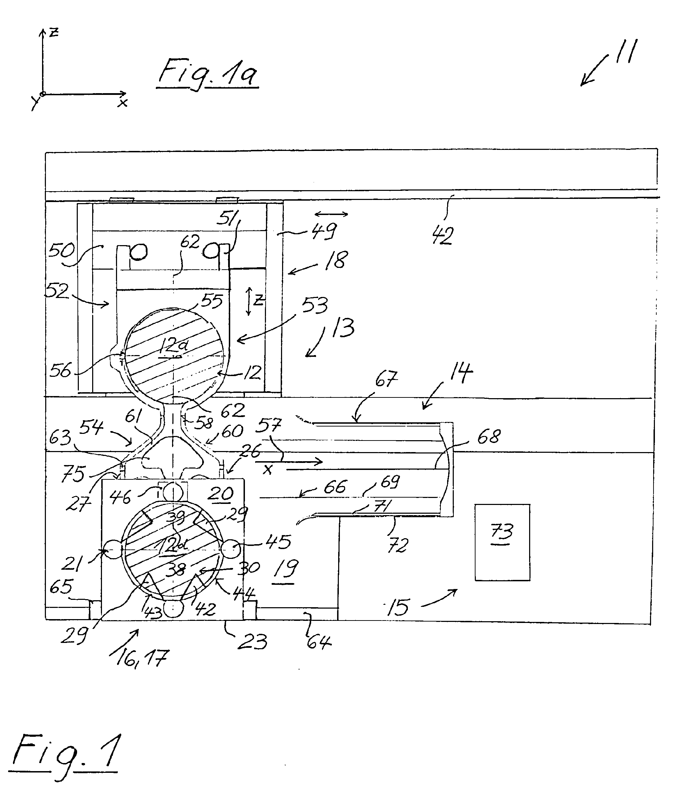

In der Fig. 1 ist ein Ausführungsbeispiel eines Automaten 11 bzw. Roboters für ein automatisches Handling von Petrischalen 12 dargestellt. Der Automat 11 umfasst eine Ent- und Beladevorrichtung 13, eine Deckelabnehmeinrichtung 14 und eine Bildauswerteeinrichtung 15 für Petrischalen 12. Es kann ein Automatengehäuse vorgesehen sein, das die Ent- und Beladevorrichtung 13, die Deckelabnehmeinrichtung 14 und die Bildauswerteeinrichtung 15 umschließt.

Die Ent- und Beladevorrichtung 13 dient zur Durchführung des erfindungsgemäßen Verfahrens zum Ent- und Beladen wenigstens eines mit einer Petrischale 12 bestückbaren Magazins. Die Ent- und Beladevorrichtung 13 umfasst ein mit wenigstens einer Petrischale 12 bestücktes Entlademagazin 16, ein Belademagazin 17, gegebenenfalls weitere Magazine, eine Transporteinrichtung 18 und eine Positioniereinrichtung 19.

Im beschriebenen Ausführungsbeispiel besitzt die Petrischale 12 ein Unterteil 12a mit Boden 12b und Seitenrand 12c sowie einen Deckel 12d mit das Unterteil übergreifendem Deckelrand 12e. Die Petrischale 12 ist vorzugsweise aus fotolichtdurchlässigem Plexiglas hergestellt. Das Unterteil 12a der Petrischale 12 dient zur Aufnahme eines Nährmediums für biologisches Material und des biologischen Materials selber. Als Nährmedium kann beispielsweise Aggar verwendet werden. Das Unterteil 12a besitzt einen kreisrunden Querschnitt. Der Deckel 12d besitzt ebenfalls einen kreisrunden Querschnitt und liegt lose auf dem Unterteil 12a auf. Im beschriebenen Ausführungsbeispiel ist der Innendurchmesser des Deckelrandes 12e größer als der Außendurchmesser des Unterteils 12a. Das Verhältnis des Deckelinnendurchmessers zum Unterteilaußendurchmessers liegt vorzugsweise im Bereich von 1,03 bis 1,05. Der Seitenrand 12c des Unterteils wird also von dem nach unten ragenden Deckelrand 12e des Deckels 12d überlappt. Der Deckelrand reicht ungefähr bis zur halben Höhe des Seitenrandes 12c herunter. Der Boden 12b der Petrischale ist eben ausgebildet. Das Verhältnis der Gesamthöhe der Petrischale 12; d.h. das Unterteil 12a mit aufgelegtem Deckel 12d; zum Deckelaußendurchmesser liegt vorzugsweise im Bereich von 0,1 bis 0,3. Das Gesamtgewicht der Petrischale 12 samt Nährmittel und biologischem Material ist so groß, dass ein nachfolgend noch näher beschriebener Ent- und Beladevorgang eines mit Petrischalen bestückbaren Magazins durchführbar ist.

Es können auch mehrere Entlade- bzw. Belademagazine 16, 17 vorgesehen sein.

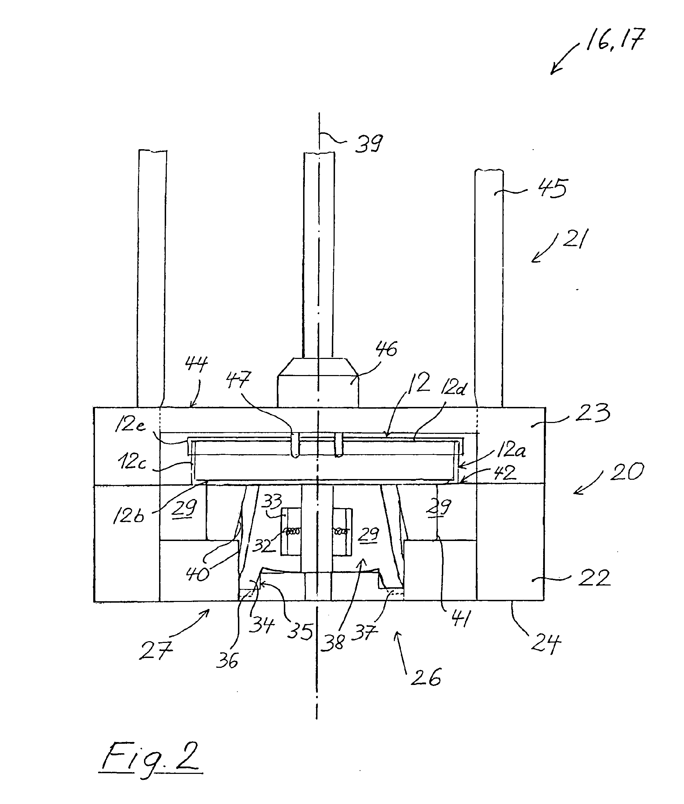

In Fig. 2 ist ein Entlade- bzw. Belademagazin 16, 17 dargestellt. Das Belademagazin 17 ist vorzugsweise identisch mit dem Entlademagazin 16, so dass die Ausführungen die sich auf das Entlademagazin 16 beziehen auch für das Belademagazin 17 gelten. Das Belademagazin 17 befindet sich vorzugsweise neben dem Entlademagazin 16 im Automat 11.

Das Entlademagazin 16 besitzt eine Basis 20 und eine seitliche Stapelführung 21.

Die Basis 20 besteht aus einem Unterteil 22 und einem Oberteil 23. Die Basis 20 ist insbesondere als massiver Metallblock, insbesondere aus Stahl oder Aluminium, hergestellt.

Das Unterteil 22 hat einen Magazinboden 24 mit Bodenöffnung 25, einer Vorderseite 26 mit Seitenöffnung 27, die unter anderem zur Entnahme von Petrischalen dient, und eine am Magazinboden 24 ausgebildete Nut 28 auf der von der Seitenöffnung abgewandten Seite des Magazins.

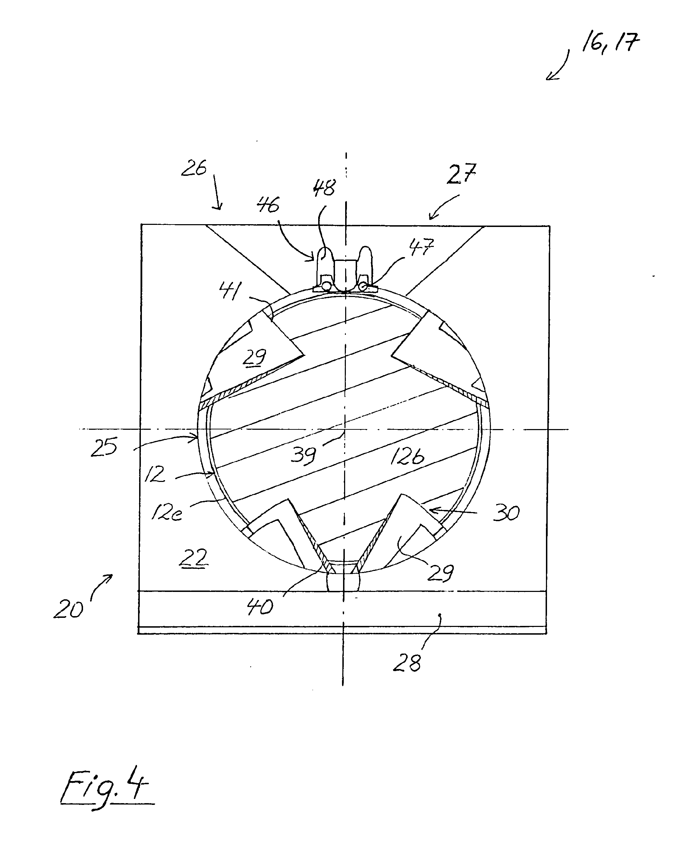

Die Fig. 4 zeigt die Unteransicht des Entlademagazins mit der Bodenöffnung 25. Die Bodenöffnung 25 hat einen kreisrunden Querschnitt und ist zur Vorderseite 26 hin trichterförmig geöffnet. Der Durchmesser der Bodenöffnung 25 ist größer als der Außendurchmesser des Deckels 12d der Petrischale 12. Es sind auch andere Querschnitte der Bodenöffnung 25 möglich, beispielsweise ein rechteckiger, quadratischer o.dgl. jedenfalls muss der Öffnungsquerschnitt der Bodenöffnung 25 größer sein als der Außendurchmesser des Petrischalen-Deckels 12d oder eines nachfolgend näher beschriebenen Supports 54, damit die Petrischale 12 bzw. der Support 54 von unten her ins Magazin gelangen kann. Die Seitenöffnung 27 hat einen rechteckigen Querschnitt. Auch hier sind andere Querschnitte möglich. Die Breite der Seitenöffnung 27 ist größer als der Außendurchmesser des Petrischalen-Deckels 12d. Die Höhe der Seitenöffnung 27 ist größer als die Gesamthöhe der Petrischale 12, damit die Petrischale 12 seitlich entnommen werden kann. Die Nut 28 verläuft parallel zur Seitenöffnung 27 vorzugsweise über die gesamte Breite des Magazinbodens 24.

Am Basis-Unterteil 22 des Entlademagazins 16 sind, der Bodenöffnung 25 zugeordnet, Sperren in Form von Klinken 29 befestigt. Die Klinken 29 sind schwenkbar zwischen einer Schließposition 30 und einer Öffnungsposition 31 gelagert. In der Schließposition 30 ragen die Klinken 29 teilweise in den Öffnungsquerschnitt der Bodenöffnung 25 hinein und bilden ihrerseits eine von der Kreisform abweichende Durchlassöffnung 38. In der Öffnungsposition 31 befinden sich die Klinken 29 nach außen zurückgedrückt zumindest teilweise in einem imBasis-Unterteil 22 gebildeten Hohlraum und geben die Bodenöffnung 25 frei. Es sind Rückstellelemente 32 in Form von Federn, insbesondere Zugfedern vorgesehen, die ein selbsttätiges Zurückschnappen der Klinken 29 von ihrer Öffnungsposition 31 in die Schließposition 30 ermöglichen. Vorzugsweise ist jeder Klinke 29 eine solche Zugfeder zugeordnet. Die Zugfedern sind einerseits mit dem Basis-Unterteil 22 und andererseits mit einem an der Klinke 29 ausgebildeten Bolzen 33 verbunden. An der Unterseite der Klinken 29 sind Führungsnasen 34 vorgesehen, die bei der Klinken-Verschwenkung zwischen der Schließposition 30 und der Öffnungsposition 31 in eine kreisbogenförmig ausgebildete Führungsbahn 35 am Basis-Unterteil 22 eingreifen. An der Vorderseite, der Bodenöffnung 25 zugewandten Seite, der Führungsbahn 35 ist ein Anschlag 36 in Form eines Steges ausgebildet, der mit einem an der Führungsnase 34 ausgebildeten Klinkenanschlag 37 zusammenwirkt und damit die Schließposition 30 der Klinke 29 vorgibt. Die Durchlassöffnung 38 der Klinken 29 verjüngt sich in axialer Richtung zu einer Magazinlängsachse 39 weg von der Bodenöffnung 25. Der lichte Durchmesser der Durchlassöffnung 38 ist kleiner als der Außendurchmesser des Deckels 12d der Petrischale 12.

Es sind vorzugsweise vier Klinken 29 vorgesehen, von denen sich zwei im Bereich der Seitenöffnung 27 und zwei insbesondere gegenüberliegend im hinteren Bereich des Basisunterteils 22 befinden. Die Klinken 29 besitzen axial zur Magazinlängsachse 39 ausgerichtete Einlaufschrägen 40. Die im Bereich der Seitenöffnung 27 angeordneten Klinken 29 besitzen zusätzlich eine Auslaufschräge 41 für das seitliche Entnehmen der Petrischale 12. Die Klinken 29 besitzen eine im wesentlichen senkrecht zur Magazinlängsachse 39 ausgerichtete ebene Oberseite 42. Die Oberseiten 42 haben in Schließposition 30 der Klinken 29 eine Dreiecksform oder die Form unregelmäßiger Vierecke. Die Oberseiten 42 bilden zusammen eine Auflagefläche 43, auf der die unterste Petrischale 12 des Entlademagazins 16 aufliegt.

Das Basisoberteil 23 ist einstückig mit dem Basisunterteil 22 verbunden. An seiner Vorderseite befindet sich der obere Teil der Seitenöffnung 27.

Das Basisoberteil 23 besitzt ferner eine Oberteilöffnung 44, die in entgegengesetzt zur Bodenöffnung 25 liegt. Die Oberteilöffnung 44 hat einen kreisrunden Querschnitt. Vorzugsweise entspricht der lichte Durchmesser der Oberteilöffnung 44 dem lichten Durchmesser der Bodenöffnung 25.

Die seitliche Stapelführung 21 dient zur seitlichen Fixierung des Petrischalenstapels im Entlademagazin 16. Als Stapelführung 21 sind im beschriebenen Ausführungsbeispiel insbesondere vier vorzugsweise in regelmäßigen Abständen um die Oberteilöffnung 44 angeordnete Stäbe 45 mit vorzugsweise kreisrundem Querschnitt vorgesehen. Durch die Höhe der Stäbe 45 wird die Gesamtstapelhöhe der Petrischalen 12 im Entlademagazin 16 vorgegeben. Die Stäbe 45 ragen teilweise in den Öffnungsquerschnitt der Oberteilöffnung 44 hinein. Der hineinragende Teil der Stäbe ist dabei als Einlaufschräge von unten ausgebildet. Der an der Vorderseite der Basis 10 oberhalb der Seitenöffnung 27 angeordnete Stab 45 hat an seiner Unterseite einen Basisabschnitt 46. Der Basisabschnitt 46 hat insbesondere die Form eines Quaders und dient zur Aufnahme von Sicherungselementen 47, die die unterste Petrischale 12 gegen seitliches Herausfallen aus der Seitenöffnung 27 sichern. Die Sicherungselemente 47 ragen dafür teilweise in den Öffnungsquerschnitt der Seitenöffnung 27 hinein. Die Sicherungselemente 47 sind vorzugsweise als an einem Ende festgelegte nach unten ragende Wendelfedern ausgebildet, die beim Entnehmen der Petrischale 12 zurückgedrückt werden und sich dabei in eine an der Unterseite des Basisabschnitts 46 ausgebildete, ungefähr an die Form der Federn angepasste Ausnehmung 48 legen.

In der Fig. 1 ist schematisch die Transporteinrichtung 18 dargestellt. Die Transporteinrichtung ist Teil der Ent- und Beladevorrichtung und dient insbesondere zum Transport der Petrischale 12 vom Entlademagazin 16 zur Deckelabnehmerrichtung 14 und von dort zur Bildauswerteeinrichtung 15 und zurück in das Belademagazin 17. Alternativ sind auch andere Transportwege möglich, beispielsweise vom Entlademagazin 16 zu einer Gebrauchseinrichtung, die nicht die Bildauswerteeinrichtung sein muss und zurück zum Belademagazin 17. Die Transporteinrichtung 18 umfasst ein Fahrgestell 49 für die Bewegung der Petrischale in eine horizontale x-Richtung, ein Fahrgestell 50 für die Bewegung in eine horizontale z-Richtung und ein Fahrgestell 51 für die Bewegung in eine zur Blattebene von Figur 1 senkrechte vertikale y-Richtung (vgl. Figur 1a).

Das Fahrgestell 49 für die Bewegung in x-Richtung ist mittels Rollen auf zwei Laufschienen 42 des Automaten 11 verfahrbar. Es ist mittels eines. Antriebsriemens mit einem als Antrieb ausgebildeten Stellmotor verbunden. Das Fahrgestell 49 für die Bewegung in x-Richtung besitzt ebenfalls zwei Schienen auf denen das Fahrgestell 50 für die Bewegung in z-Richtung mittels Rollen verfahrbar ist. Auch dieses Fahrgestell ist über einen Antriebsriemen mit einem Stellmotor gekoppelt. Das Fahrgestell 50 für die Bewegung in z-Richtung besitzt zwei in vertikaler Richtung angeordnete Schienen, auf denen das Fahrgestell 51 für die Bewegung in y-Richtung mittels Rollen verfahrbar angeordnet ist. Auch dort ist ein Antriebsriemen und ein Stellmotor vorgesehen. Es ist also möglich, jede beliebig Position innerhalb des Automaten 11 anzufahren.

Das Fahrgestell 51 für die Bewegung in y-Richtung besitzt einen Tandemschlitten 52 mit einer Schiebeeinrichtung 53 und einem Support 54.

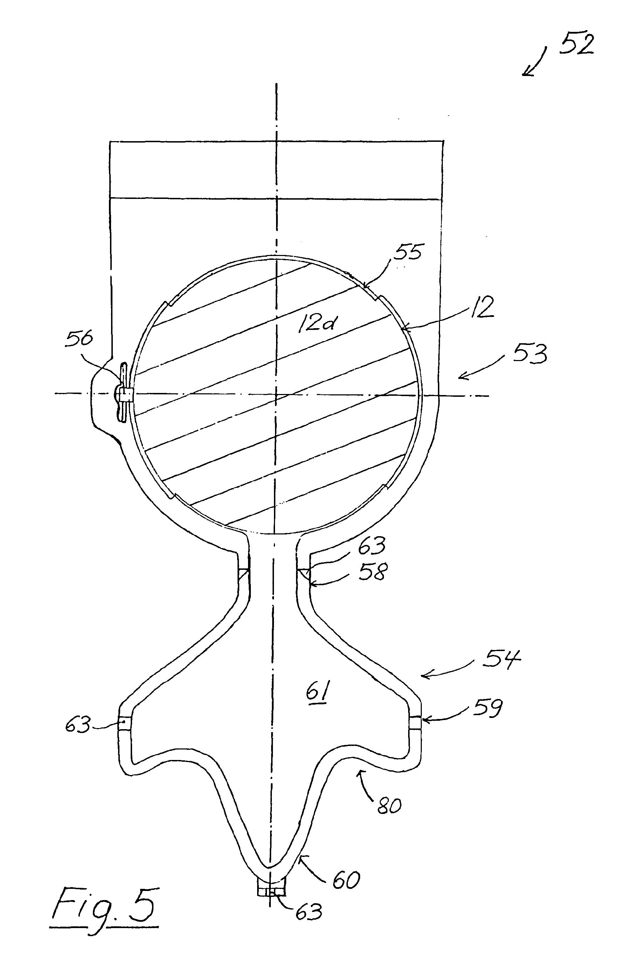

In der Fig. 5 ist der Tandemschlitten 52 dargestellt. Die Schiebeeinrichtung 53 ist im beschriebenen Ausführungsbeispiel als Ring ausgebildet. Der lichte Innendurchmesser des Rings ist größer als der Außendurchmesser des Unterteils 12a der Petrischale 12 und insbesondere an Stegbereichen 55 kleiner als der Außendurchmesser des Deckels 12d der Petrischale 12. An der Schiebeeinrichtung 53 ist ein Vorsprung 56 in Form eines Rädchens vorgesehen, das in Schieberichtung in die Querschnittsöffnung der Schiebeeinrichtung 53 hineinragt. Das Rädchen dient zum Erfassen des Deckelrandes 12e des Petrischalen-Deckels 12d. An der nicht dargestellten Unterseite der Schiebeeinrichtung 53 befindet sich ein Einschnitt, der beim Verschieben der Petrischale mit einer nachfolgend näher beschriebenen Führung für das Petrischalen-Unterteil 12a zusammenwirkt.

Der Support 54 ist einstückig und in etwa gleicher Höhe mit der Schiebeeinrichtung 53 verbunden und dient zur Aufnahme und zum Transport der Petrischale 12. Er ist als schmales Materialband, insbesondere aus - Stahl, ausgebildet, mit einer charakteristischen äußeren und inneren Kontur. Der Support 54 besitzt einen Übergangsabschnitt 58, der die Verbindung mit der Schiebeeinrichtung darstellt, einen breiten Mittelabschnitt 59, einen annähernd spitz zulaufenden Endabschnitt 60 und eine mittige Durchbrechung 61.

Der Übergangsabschnitt 58 schließt sich insbesondere unmittelbar an die Schiebeeinrichtung 53 an und ist spiegelsymmetrisch und parallel zu einer Schlittenlängsachse 62 ausgerichtet. Am Übergangsabschnitt 58 sind Zentrierelemente 63 in Form von Nasen ausgebildet, die sich in vertikaler y-Richtung, also senkrecht zur Schlittenlängsachse 62 erstrecken. Die Nasen sind in Draufsicht dreieckig und haben eine zur Schlittenlängsachse 62 geneigte Schräge. An den Übergangsabschnitt 58 schließt sich der Mittelabschnitt 59 an. Er besitzt gegenüber dem Übergangsabschnitt 58 eine größere horizontale Querabmessung senkrecht zur Schlittenlängsachse 62. Der Mittelabschnitt 59 ist ebenfalls spiegelsymmetrisch zur Schlittenlängsachse 62 ausgebildet und hat die Form von zwei aufgespreizten Flügeln. An der dem Übergangsabschnitt 58 gegenüberliegenden Seite des Mittelabschnitts 59 befinden sich, quasi an der von der Schiebeeinrichtung 53 abweisenden Seite der Flügel, Einbuchtungen 80. Der Mittelabschnitt 59 besitzt ebenfalls in y-Richtung zeigende Nasen. Der Mittelabschnitt 59 geht in den Endabschnitt 60 über. Der Endabschnitt 60 ist in etwa parabelförmig ausgebildet. Er besitzt ebenfalls eine in y-Richtung ausgerichtete Nase. Die Nasen am Übergangsabschnitt 58 am Mittelabschnitt 59 und am Endabschnitt 60 bilden eine Umgreifung für eine aufzunehmende Petrischale 12. Der lichte Durchmesser der Umgreifung ist größer als der Außendurchmesser des Petrischalen-Unterteils 12a. Der Durchmesser der Umgreifung vergrößert sich vertikaler y-Richtung, so dass beim Übernehmen der Petrischale 12 Fehlstellungen mit dem Support 54 ausgeglichen werden können.

Die Positioniereinrichtung 19 dient zum Positionieren des Entlademagazins 16 und des Belademagazins 17 im Automat 11. Die Positioniereinrichtung 19 besitzt eine horizontale Stange 64, die in die Nut 28 am Magazinboden 24 eingreifen kann. Es sind ferner Positionieranschläge 65 vorgesehen, die die genaue Position der Magazine innerhalb des Automaten 11 festlegen.

In der Fig. 1 ist ein Tisch 75 dargestellt, der beim Umsetzen der Petrischale vom Support 54 in die Schiebeeinrichtung 53 verwendet wird. Der Tisch hat eine äußere Kontur, die an die innere Kontur der mittigen Durchbrechung 61 am Support 54 angepasst ist. Die größte Querabmessung des Tisches ist dabei kleiner als die größte Querabmessung des Supports 54, so dass der Support über den Tisch abgesenkt werden kann.

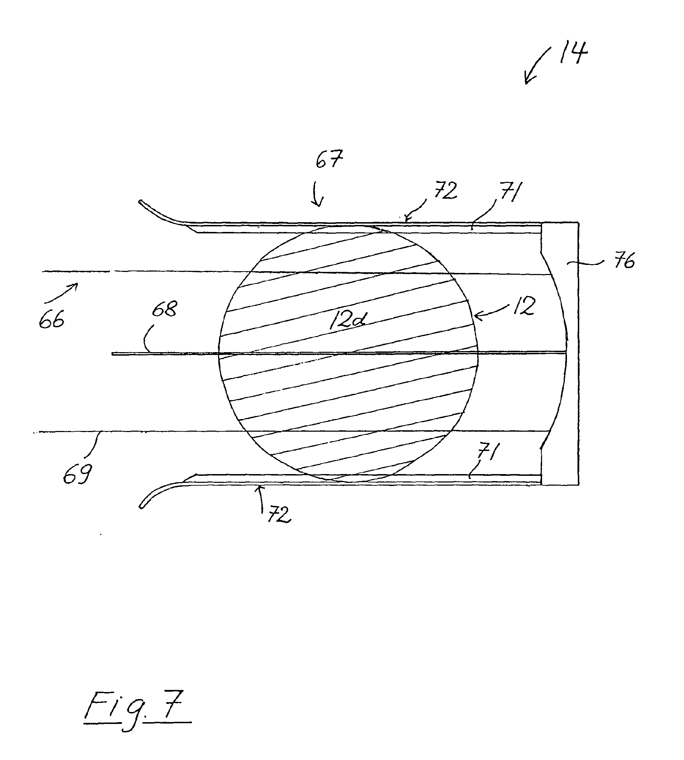

In den Fig. 1, 6 und 7 ist eine Deckelabnehmeinrichtung 14 dargestellt, die zur Durchführung des Verfahrens zum Abnehmen des Deckels einer Petrischale verwendet wird. Die Deckelabnehmeinrichtung 14 besteht im wesentlichen aus einer Unterteilführung 66, einer schräg dazu angeordneten Deckelführung 67 und einem Niederhalter 68.

Die Unterteilführung 66 ist im beschriebenen Ausführungsbeispiel als U-Profil mit zwei in vertikaler y-Richtung ausgerichteten Schenkeln 69 ausgebildet. Der lichte Abstand der beiden Schenkel 69 ist kleiner als der lichte Abstand des Einschnitts an der Unterseite der Schiebeeinrichtung 53. Somit lässt sich die Schiebeeinrichtung 53 und mit dieser die Petrischale auf die Unterteilführung 66 aufschieben. An der in Schieberichtung 57 liegenden Vorderseite der beiden Schenkel 69 ist jeweils eine Unterteilführungs-Einlaufschräge 70 ausgebildet. Der nicht dargestellte Endbereich der Unterteilführung 66 kann gegenüber der Horizontalen in Schieberichtung 57 schräg nach unten ausgerichtet sein.

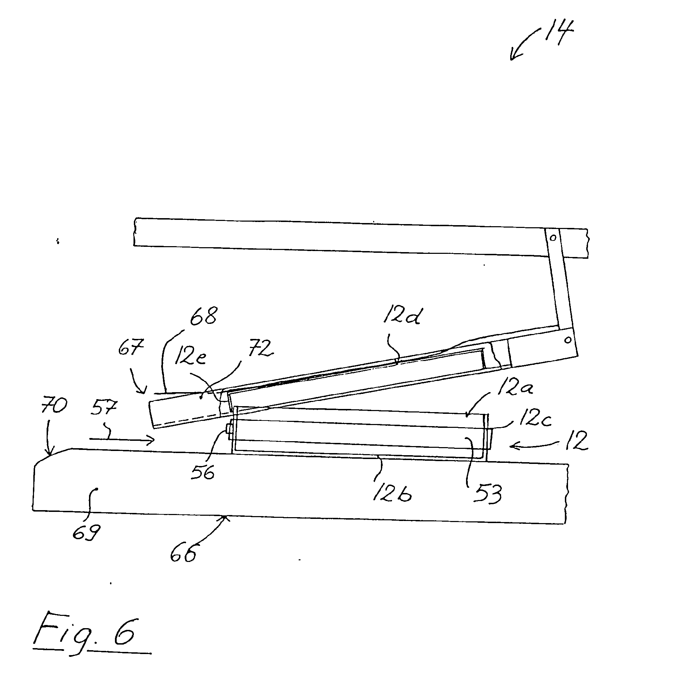

Die Deckelführung 67 besitzt eine Deckeluntergreifungseinrichtung 71 und eine Seitenführung 72. Die Deckelführung 67 besitzt eine gegenüber der Unterteilführung in Pfeilrichtung 57 (Fig. 6) nach oben ausgerichtete Schräge. Die Deckeluntergreifungseinrichtung 71 wird von zwei parallel zur Schieberichtung 57 angeordneten Schienen gebildet. Der lichte Abstand der beiden Schienen ist kleiner als der Außendurchmesser des Petrischalen-Deckels 12d und größer als der Außendurchmesser des Unterteils 12a. Die in Schieberichtung 57 liegenden Vorderkanten der Schienen sind in einer bestimmten Höhe über der Unterteilführung 66 angeordnet. Der lichte Abstand zwischen der Unterteilführung 66 und den Vorderkanten der Schienen ist kleiner als die Höhe des Seitenrandes 12c des Unterteils 12a der Petrischale 12. Somit kann das Petrischalen-Unterteil 12a bei einer der Schieberichtung 57 entgegengesetzten Bewegung mit seinem Seitenrand 12c an den Deckelrand 12e anstoßen und den Deckel 12d wieder mitnehmen.

Die Seitenführung 72 ist der Deckeluntergreifungseinrichtung 71 zugeordnet und insbesondere mit dieser einstückig verbunden. Es sind insbesondere zwei Führungselemente in Form von Seitenführungsschenkeln vorgesehen, die jeweils mit einer Schiene der Deckeluntergreifungseinrichtung 71 einstückig verbunden sind. Die Seitenführung 72 ist dazu vorgesehen, dass beim Verschieben der Petrischale 12 der Petrischalen-Deckel 12d gegenüber dem Petrischalen-Unterteil 12a in der Spur bleibt, d.h. sich der Deckel 12d und das Unterteil 12a nicht in zwei unterschiedliche Horizontalrichtungen bewegen. Der lichte Abstand zwischen den beiden Seitenführungsschenkeln entspricht ungefähr dem Außendurchmesser des Petrischalen-Deckels 12d. An den Schenkeln der Seitenführung 72 kann eine Seitenführungs-Einlaufschräge vorgesehen sein, beispielsweise in Form einer Umbiegung nach außen. Beim Verschieben der Petrischale in Pfeilrichtung wird der Deckel (12d) durch Schräglage der Deckelführung 67 vom Unterteil abgehoben, so dass das Unterteil alleine weiterbeförderbar ist.

Der Niederhalter 68 erfüllt eine Doppelfunktion. Zum einen bremst er den Deckel 12d bei seiner Bewegung in der Deckel-Führung 67, zum anderen hält er den abgehobenen Deckel 12d in einer im wesentlichen senkrecht zur Bewegungsrichtung verlaufenden Richtung nieder. Der Niederhalter 68 kann ein relativ dünner Federdraht sein, der sich insbesondere mittig zwischen den beiden Seitenführungsschenkeln befindet. Gegebenenfalls ist eine Einlaufschräge in Form einer Aufbiegung des Drahts vorgesehen.

Die Bildauswerteeinrichtung 15 dient zur Bildauswertung des auf dem Nährboden der Petrischale befindlichen biologischen Materials. Die Bildauswerteeinrichtung 15 umfasst eine Kamera 73, eine lichtdurchlässige Photolichtplatte, einen Umlenkspiegel und eine Auf- und/oder Durchlichteinheit. Mit der Kamera 73 können Farbbilder und/oder Schwarz-Weiß-Bilder vom biologischen Material erstellt werden. Die Kamera 73 ist vorzugsweise mit einem Bildschirm eines Auswerterechners gekoppelt sein, so dass sofort ausgewertet werden kann. Die lichtdurchlässige Photolichtplatte befindet sich bevorzugt benachbart zur Unterteilführung 66 der Deckelabnehmeinrichtung 14, so dass das Unterteil 12a von der Unterteilführung 66 auf die Photolichtplatte gefahren werden kann. Die Auflichteinheit besitzt eine Ringleuchte und eine über der Ringleuchte angeordnete Abschirmplatte zur Lichtabschirmung nach oben.

### Verfahren

Durch die Ent- und Beladevorrichtung 13, insbesondere durch deren Transporteinrichtung 18, lässt sich die unterste Petrischale 12 aus dem Entlademagazin 16 herausnehmen und nach Gebrauch in das Belademagazin 17 zurücklegen.

Wie insbesondere in Fig. 1 dargestellt, fährt zunächst das Fahrgestell 49 für die Bewegung in x-Richtung zur Position der Positioniereinrichtung 19, in der sich das Entlademagazin 16 befindet. Das Fahrgestell 50 zur Bewegung in z-Richtung fährt auf die Positioniereinrichtung 19 zu, bis der Support 54 des Tandemschlittens 52 des Fahrgestells 51 für die Bewegung in y-Richtung unterhalb der Basis 20 des Entlagemagazins 16 im wesentlichen fluchtend zur Bodenöffnung 25 ausgerichtet ist.

Als nächstes fährt das Fahrgestell 51 für die Bewegung in y-Richtung hoch. Dabei wird der Tandemschlitten 52 nach oben bewegt und der Support 54 fährt durch die Bodenöffnung 25 in das Entlademagazin 16 ein. Die einstückig mit dem Support 54 verbundene Schiebeeinrichtung 53 hingegen bleibt außerhalb des Entlademagazins 16. Der Support 54 gelangt von der Bodenöffnung 25 nach oben in die von den Klinken 29 gebildete Durchlassöffnung 38. Beim Einfahren drückt dabei der Mittelabschnitt 59 mit seiner großen Querabmessung die vorderen, im Bereich der Seitenöffnung 27 angeordneten Klinken 29 von ihrer Schließposition 30 nach außen in ihre Öffnungsposition 31. Je weiter der Support 54 nach oben fährt, desto weiter werden die Klinken 29 nach außen zurückgedrückt. Dies wird durch die Einlaufschrägen 40 der Klinken 29 erreicht. Die Klinken 29 bewegen sich dabei mit ihrer kreisbogenförmigen Führungsnase 34 auf der Führungsbahn 35 des Basis-Unterteils 22.

Der Support 54 wird so lange nach oben gefahren, bis er eben mit den durch die Oberseiten 42 der Klinken 29 gebildeten Auflagefläche 43 abschließt. Der Boden 12d des Petrischalen-Unterteils 12a wird vom Support 54 erfasst, und die Petrischale 12 liegt nunmehr sowohl auf der Auflagefläche 43 der Klinken 29 als auch auf dem Support 54 auf. Vorher schon umgreifen die nasenförmigen Zentrierelemente 63 den Seitenrand 12c des Petrischalen-Unterteils 12a, so dass eine etwaige Fehlstellung zwischen der Petrischale 12 und dem Support 54 ausgeglichen wird.

Als nächstes wird das Fahrgestell 50 für die Bewegung in z-Richtung zurückgefahren, so dass der Support 54 mitsamt der untersten Petrischale 12 seitlich aus der Seitenöffnung 27 herausgefahren wird. Dabei werden durch den Mittelabschnitt 59 des Supports 54 die vorderen Klinken 29 noch weiter zurückgedrückt, so dass der Support 54 ausfahren kann. Die in den Öfnungsquerschnitt der Seitenöffnung 27 ragenden federförmigen Sicherungselemente 47 werden dabei durch den Petrischalen-Deckel 12d nach außen zurückgebogen, so dass sie das seitliche Entnehmen nicht behindern.

Sobald der Mittelabschnitt 59 des Supports 54 vollständig ausgefahren ist, schnappen die vorderen Klinken 29 von ihrer Öffnungsposition 31 zurück in ihre Schließposition 30. Dabei schlägt der an den Führungsnasen 34 der Klinken 29 befindliche Klinkenanschlag 37 an den Anschlag 36 an der Vorderseite der kreisbogenförmigen Führungsbahn 35 an und die Klinken 29 sind in ihrer Schließposition 30 fixiert. Der im Magazin verbleibende Stapel rutscht nach unten, so dass die jetzt unterste Petrischale auf die Oberseite der Klinken zu liegen kommt.

Der Support 54 wird so weit zurückgefahren, bis er über dem Tisch 75 ausgerichtet ist. Der Tisch 75 wird fluchtend zur mittigen Durchbrechung 61 des Supports 54 ausgerichtet. Das Fahrgestell 51 zur Bewegung in y-Richtung mitsamt dem Tandemschlitten 52 wird nach unten bewegt bis der Tisch 75 durch die Relativbewegung in die Durchbrechung 61 am Support 54 einfährt und dabei den Boden des Petrischalen-Unterteils 12a erfasst. Die Petrischale 12 ist nun auf dem Tisch 75 abgestellt. Als nächstes fährt das Fahrgestell 50 nach vorne, bis die kreisringförmige Schiebeeinrichtung 57 unterhalb des Tisches 75 liegt, wobei eine Öffnung zwischen den Ringen der Schiebeeinrichtung 53 und des Supports 54 ein Umfahren eines Tischbeins ermöglicht. Das Fahrgestell 51 und der Tandemschlitten 52 fahren nach oben. Da der lichte Durchmesser der Schiebeeinrichtung 53 größer ist als der Außendurchmesser des Petrischalen-Unterteils 12a wird das Petrischalen-Unterteil 12a nicht erfasst, sondern die Schiebeeinrichtung 53 fährt weiter, bis der Petrischalen-Deckel 12d durch Stegbereiche 55 der Schiebeeinrichtung erfasst wird. Das Petrischalen-Unterteil 12a bleibt auf dem Tisch 75 abgestellt. Der an der Schiebeeinrichtung 57 ausgebildete Vorsprung 56 in Form des Rädchens greift an den Deckelrand 12e des Petrischalendeckels 12d an und die Petrischale 12 kann seitlich verschoben werden.

Als nächstes wird das Fahrgestell 49 seitlich verschoben und gibt dabei die Schieberichtung 57 vor. Dabei wird die Petrischale 12 am Deckel 12e seitlich verschoben, so dass sie zunächst vom Tisch 75 auf die Unterteilführung 66 der Schiebeeinrichtung 53 gelangt. Das Petrischalen-Unterteil 12a liegt dabei auf der Unterteilführung 66 auf. Ein weiteres seitliches Verschieben führt dazu, dass die Deckelführung 67 erreicht wird. Die Deckeluntergreifungen 71 untergreifen die Unterkante des Deckelrandes 12e und der Deckel wird leicht angehoben und an der in Schieberichtung 57 weisenden Seite relativ vom Unterteil 12a weggekippt bzw. weggeschwenkt. Durch die Steigung der Deckelführung 67 in Schieberichtung 57 kommt es dazu, dass bei einer weiteren seitlichen Verschiebung der Öffnungswinkel zwischen dem Deckel und dem Unterteil 12a immer größer wird. Der Deckel 12d wird dabei durch die Deckeluntergreifungen 71 und seitlich durch die Seitenführungen 72 geführt und bleibt fluchtend zum Unterteil 12a ausgerichtet. Nach einem bestimmten Schiebeweg und Erreichen des Winkels zwischen Deckelführung 67 und Unterteilführung 66 wird der Deckel von Schiebevorsprung 56 abgehoben und durch den Niederhalter 68 abgebremst, so dass er nicht mehr weiter in Schieberichtung 57 seitlich verschoben werden kann. Der Deckelrand 12d ist an dieser Stelle jedoch an der in Schieberichtung 57 hinteren Seite der Petrischale 12 mit dem Seitenrand 12c des Petrischalen-Unterteils 12a noch überlappend in Kontakt.

Als nächstes wird das Fahrgestell 51 zur Bewegung in y-Richtung nach unten gefahren, so dass der Vorsprung 56, insbesondere das Rädchen, der Schiebeeinrichtung 53 jetzt am Seitenrand 12c des Petrischalen-Unterteils 12a zur Anlage kommt. Das Petrischalen-Unterteil 12a wird dann seitlich weitergeschoben, der Deckel 12d jedoch bleibt auf der Deckelführung 67 liegen. Das Petrischalen-Unterteil 12a wird mittels der Schiebeeinrichtung 53 in den Bereich der Bildauswerteeinrichtung 15 geschoben. Dabei gelangt das Petrischalen-Unterteil 12a von der Unterteilführung 66 direkt auf die lichtdurchlässige Photolichtplatte. Das Petrischalen-Unterteil 12a wird von unten durchleuchtet und von oben mittels der Ringleuchte angestrahlt. Es wird mittels der Kamera wenigstens ein Bild des auf dem Nährboden befindlichen biologischen Materials, beispielsweise von Keimen, Bakterien oder dergleichen aufgenommen. Als nächstes erfolgt eine Auswertung anhand der Bilder.

Nach der Bildauswertung wird das Petrischalen-Unterteil 12a durch die Schiebeeinrichtung 53 entgegen der ursprünglichen Schieberichtung 57 zurück in den Bereich der Deckelabnehmeinrichtung 14 geschoben. Der Seitenrand 12c des Petrischalen-Unterteils 12a erfasst den Deckelrand 12e des Petrischalen-Deckels 12d, so das der Deckel 12d mitgenommen wird und wieder lose auf dem Petrischalen-Unterteil 12a zu liegen kommt.
Als nächstes erfolgt ein Umsetzen der Petrischale 12 von der Schiebeeinrichtung 57 auf den Tisch 75 und wird dann wieder von dem Support 54 übernommen analog der zuvor beschriebenen Weise.

Das Fahrgestell 49 fährt auf die Höhe des Belademagazins 17. Als nächstes fährt das Fahrgestell 50 nach vorne, so dass der Support mitsamt der Petrischale 12 unterhalb der Bodenöffnung 25 des Belademagazins 17 zu liegen kommt. Das Fahrgestell 51 mitsamt dem Tandemschlitten 52 fährt nach oben, so dass die Petrischale 12 und der Support 54 durch die Bodenöffnung 25 in das Belademagazin 16 gelangen. Von der Bodenöffnung 25 gelangt die Petrischale 12 in die durch die Klinken 29 gebildete Durchlassöffnung 38. Dabei werden alle vier Klinken 29 durch den Deckel 12d der Petrischale 12 von ihrer Schließposition 30 in ihre Öffnungsposition 31 gedrückt. Befinden sich im Belademagazin 17 Petrischalen, werden diese durch die eingeführte Petrischale 12 nach oben gedrückt. Der Support 54 mitsamt der Petrischale 12 fährt so lange nach oben, bis er eben mit der durch die Klinken 29 gebildeten Auflagefläche 43 abschließt. Dabei schnappen die Klinken 29 ein bestimmtes Stück in Richtung Schließposition 30 bis zum Anschlag an den Seitenflächen des Support zurück, so dass die eingeführte Petrischale 12 untergriffen wird. Das Fahrgestell 51 fährt nach unten, so dass der Support 54 aus dem Belademagazin 17 herausfährt. Dabei schnappen die Klinken 29 vollständig in ihre Schließposition 30 zurück. Die Petrischale 12 ist im Belademagazin 17 abgelegt.

## Patentansprüche

1. Verfahren zum Abnehmen eines Deckels einer Petrischale, wobei die Petrischale ein einen Boden und eine zylindrische Seitenwand aufweisendes schalenförmiges Unterteil besitzt und der einen nach unten weisenden Deckelrand, die Seitenwand des Unterteils außen überlappende Deckel lose auf dem Unterteil aufliegt, das Verfahren mit folgenden Schritten:
- Erfassen des Deckelrandes an seiner Unterkante an mindestens zwei gegenüberliegenden Seiten;
- Seitliche Bewegung des Unterteils und des Deckels auf getrennten Führungen, die in Bewegungsrichtung unter relativem Anheben des Deckels in einem divergierenden Winkel zueinander verlaufen, wobei der Deckel an der in Bewegungsrichtung weisenden Seite relativ vom Unterteil unter Kippbewegung soweit angehoben wird, dass das Unterteil unter dem Deckel wegbewegbar ist;
- Relatives Wegbewegen des Unterteils vom Deckel.

2. Verfahren nach Anspruch 1, **dadurch gekennzeichnet, dass** der Deckel beim Anheben in einer zu einer Bewegungsrichtung des Deckels im wesentlichen senkrechten Richtung insbesondere federnd niedergehalten wird.

3. Verfahren nach einem der vorhergehenden Ansprüche, **dadurch gekennzeichnet, dass** die Petrischale bei der seitlichen Bewegung am Deckel erfasst wird und, vorzugsweise nur, am Deckel seitlich verfahren, insbesondere verschoben wird, wobei die Petrischale vorzugsweise bei einem bestimmen Öffnungswinkel zwischen Deckel und Unterteil am Unterteil erfasst wird und am Unterteil seitlich verfahren, insbesondere verschoben wird.

4. Verfahren nach einem der vorhergehenden Ansprüche, **dadurch gekennzeichnet, dass** der Deckel in einer solchen relativen Kipplage verbleibt, dass das Unterteil bei einer Rückwärtsbewegung den Deckel mitnimmt, so dass dieser bei Rückgängigmachung der relativen Kipplage wieder auf dem Unterteil zur Ablage kommt.

5. Verfahren nach einem der vorhergehenden Ansprüche, **dadurch gekennzeichnet, dass** das Unterteil nach Abnehmen des Deckels in eine Position zum Gebrauch, insbesondere zur Bildauswertung gebracht und danach vorzugsweise wieder in Richtung zum Deckel bewegt wird, wobei der Deckel wieder aufgelegt wird.

6. Deckelabnehmeinrichtung zum Abnehmen eines Deckels (12d) einer Petrischale (12), wobei die Petrischale (12) ein einen Boden (12b) und einen zylindrischen Seitenrand (12c) aufweisendes schalenförmiges Unterteil (12a) besitzt und der einen nach unten weisenden Deckelrand (12e), den Seitenrand (12c) des Unterteils (12a) außen überlappende Deckel (12d) lose auf dem Unterteil (12a) aufliegt, mit einer Deckelführung (67) zum Anheben des Deckels (12d) vom Unterteil (12a) und einer Unterteilführung (66), die in Bewegungsrichtung und Anhebrichtung in einem divergierenden Winkel zueinander angeordnet sind.

7. Deckelabnehmeinrichtung nach Anspruch 6, **dadurch gekennzeichnet, dass** die Deckelführung (67) eine Schräge aufweist, die in Bewegungsrichtung gegenüber der Horizontalen ansteigend ausgerichtet ist.

8. Deckelabnehmeinrichtung nach Anspruch 6 oder 7, **dadurch gekennzeichnet, dass** die Deckelführung (67) als Deckeluntergreifungseinrichtung (71) zur seitlichen Untergreifung des Deckelrandes (12e) ausgebildet ist und vorzugsweise ein der Petrischale (12) zugewandtes Ende der Deckeluntergreifungseinrichtung (71) unterhalb einer Unterkante des Deckelrandes (12c) angeordnet ist, insbesondere als Deckeluntergreifungseinrichtung (71) zwei Schienen vorgesehen sind, die im wesentlichen parallel zur Bewegungsrichtung beabstandet voneinander angeordnet sind, wobei deren lichter Abstand kleiner ist als der Außendurchmesser des Deckels (12d) und größer ist als der Außendurchmesser des Unterteils (12a).

9. Deckelabnehmeinrichtung nach einem der Ansprüche 6 bis 8, **dadurch gekennzeichnet, dass** der Deckeluntergreifungseinrichtung (71) eine Seitenführung (72) zur seitlichen Führung des Deckels (12d) zugeordnet ist, sie insbesondere eine solche aufweist, wobei vorzugsweise die Seitenführung (72) zwei Führungselemente, aufweist, die parallel zur Bewegungsrichtung beabstandet voneinander angeordnet sind, wobei deren lichter Abstand im wesentlichen dem Außendurchmesser des Deckels (12d) entspricht.

10. Deckelabnehmeinrichtung nach einem der Ansprüche 6 bis 9, **dadurch gekennzeichnet, dass** die Deckelführung (67) eine Bremse und/oder einen Anschlag (76) zur Begrenzung der Deckelbewegung in Bewegungsrichtung, insbesondere in Schieberichtung (57), aufweist, wobei vorzugsweise die Bremse und/oder der Anschlag so angeordnet sind, dass in der Endlage des Deckels (12d), der lichte Abstand zwischen der Deckeluntergreifungseinrichtung (71), an einer in Bewegungsrichtung hinteren Seite des Deckels (12d) und der Unterteilführung (66) kleiner ist als die Höhe des Seitenrandes (12c) des Unterteils (12a).

11. Deckelabnehmeinrichtung nach einem der Ansprüche 6 bis 10, **dadurch gekennzeichnet, dass** eine Gebrauchseinrichtung zum Gebrauch der Petrischale (12) vorgesehen ist, die vorzugsweise eine Bildauswertungseinrichtung (15) ist und wobei insbesondere die Unterteilführung (66) zur Gebrauchseinrichtung führt, wobei vorzugsweise die Gebrauchseinrichtung eine Bildauswerteeinrichtung (15) ist, insbesondere mit einer lichtdurchlässigen Fotolichtplatte, einer Kamera (73) und einer Auf- und oder Durchlichteinheit.

12. Deckelabnehmeinrichtung nach einem der Ansprüche 6 bis 11, **dadurch gekennzeichnet, dass** wenigstens ein Fahrgestell (49, 50, 51) zur Bewegung der Petrischale (12) vorgesehen ist und das Fahrgestell (49, 50, 51) vorzugsweise mit einem Schlitten zum Transport der Petrischale (12) gekoppelt ist, wobei der Schlitten insbesondere als Tandemschlitten (52) ausgebildet ist, mit einem Support (54) zur Aufnahme der Petrischale (12) und einer Schiebeeinrichtung (53) zum seitlichen Erfassen und Verschieben der Petrischale (12).

13. Deckelabnehmeinrichtung nach Anspruch 12, **dadurch gekennzeichnet, dass** der Support (54) eine mittige Durchbrechung (61) aufweist, insbesondere der Support (54) als relativ schmales Materialband ausgebildet ist, das eine äußere und eine die Durchbrechung (61) definierende innere Kontur bildet.

14. Deckelabnehmeinrichtung nach Anspruch 12, **dadurch gekennzeichnet, dass** die Schiebeeinrichtung (53) in Schieberichtung (57) gegenüberliegenden Randbegrenzungen insbesonere Zentrierungen aufweist, deren lichter Abstand größer ist als der Außendurchmesser des Unterteils (12a) und kleiner ist als der Außendurchmesser des Deckels (12d), wobei im Bereich der Randbegrenzungen vorzugsweise ein Vorsprung (56) vorgesehen ist, der in Schieberichtung (53) den Abstandsbereich der Randbegrenzungen hineinragt.

15. Deckelabnehmeinrichtung nach einem der Ansprüche 6 bis 14, **dadurch gekennzeichnet, dass** als Bremse ein Niederhalter (38) zum Niederhalten des Deckels (12d) bei dessen Anhebung gegenüber dem Unterteil (12a) vorgesehen ist.

16. Automat zum Handling von Petrischalen (12) mit einer Deckelabnehmeinrichtung (14) nach einem der Ansprüche 6 bis 15 und einer Ent- und Beladevorrichtung (13) für Petrischalen (12).
